# EUROPEAN PATENT SPECIFICATION

(11) **EP 3 520 893 B1**
(45) Date of publication and mention of the grant of the patent: **22.07.2020**
(21) Application number: 18154962.7
(22) Date of filing: 02.02.2018
(51) Int. Cl.: B01L 3/00, B01L 9/00

(54) **SYSTEM FOR THE THERMALLY CONTROLLED PROCESSING OF A BIOLOGICAL SAMPLE**
SYSTEM ZUR THERMISCH GESTEUERTEN VERARBEITUNG EINER BIOLOGISCHEN PROBE
SYSTÈME POUR LE TRAITEMENT COMMANDÉ THERMIQUEMENT D'UN ÉCHANTILLON BIOLOGIQUE

(43) Date of publication of application: 07.08.2019
(73) Proprietor: F. Hoffmann-La Roche AG, 4070 Basel (CH); Roche Diagnostics GmbH, 68305 Mannheim (DE)
(72) Inventor: BERNET, Roland, CH-6343 Rotkreuz (CH); CHERUBINI, Claudio, CH-6343 Rotkreuz (CH); HERMANN, Pius, CH-6343 Rotkreuz (CH); SAROFIM, Emad, CH-6343 Rotkreuz (CH)
(74) Representative: Merkel, Patrick

(56) References cited:
- EP-A1- 2 618 157
- WO-A1-2011/047233
- US-B1- 6 368 561

## Description

### Field of the Invention

The present invention belongs to the field of systems and methods for conducting biological or biochemical assays. Within this field, it relates to thermally controlled processing of a liquid biological sample, such as a sample containing biomolecules, with the aid of magnetic particles employed in a system.

### Background

Processing of biological samples such as clinical samples in analytical applications often involves the use of magnetic micro- or nanoparticles, especially in the field of clinical diagnostics. Downstream analytical methods for target molecules isolated using magnetic particles such as binding particles are, for example, amplification, detection and/or sequencing of nucleic acids such as RNA, DNA, mRNA (by means of PCR or other amplification techniques), mass spectrometry, ELISA or electro- or chemiluminescence assays for proteins, next generation sequencing (NGS), and the like.

Typically, the magnetic particles exhibit a surface to which a biological target material can be bound using appropriate conditions. In contrast to non-magnetic binding particles, they can be retained by a magnetic field gradient when withdrawing the respective supernatant during an isolation process for a biological target material, thus abolishing the need for centrifugation or comparable techniques.

Sample preparation techniques based on the use of magnetic particles are well known in the art and appreciated for their ease of automation as well as other advantages.

However, it has proven difficult to efficiently conduct the process in small volumes contained in a relatively high number of compartments of a reaction vessel, such as a multiwell plate, particularly in cases where there is a need for thermally controlling the sample to be processed. In general, miniaturization of sample volume is often desirable as it tends to increase throughput and parallelization. However, such miniaturization usually requires a reduction of size and/or complexity of the involved hardware, which may be technically demanding.

A further challenge in the art has been to conduct the process reliably, unattended and fully automated, as well as to process several samples in parallel.

Efficient binding of analytes to the surface of magnetic particles, as well as related preparation steps such as washing or elution, are often temperature-dependent, which is why thermal control of the respective process can be of considerable importance. Therefore, there is a need in the art for improved systems and methods for the magnet-based processing of biological samples under thermal control.

One approach in the art, as disclosed in US6368561 B1, relies on the spatial and temporal separation of magnetic attraction on the one hand and thermal incubation on the other hand. In brief, this document teaches the skilled person to use a holder for multiple vessels that in one step of the processing method interacts with a magnetic arrangement, and is then relocated to a hotplate via a horizontal transfer system, or vice versa. Consequently, the footprint of such a system is at least large enough to support the footprint of the magnetic arrangement as well as the footprint of the hotplate.

The present disclosure describes an approach that avoids various shortcomings in the art.

### Summary

In a first aspect described herein, a system for processing a biological target material from a liquid sample is provided. The system includes a multiwell plate with a suspension of magnetic particles, a magnetic separation device with a magnetic separation plate, and a heating device with a non-magnetic heating adapter. The heating adapter has both receptacles (on its upper face) for the wells of the multiwell plate and recesses (in its lower face) for the magnets of the magnetic separation plate. The distance between the receptacles and the recesses is sufficiently small for the inserted magnets to impose a magnetic field gradient onto the wells of the multiwell plate. Heating is provided by a heating element between the receptacles on the upper face of the heating adapter.

In another aspect, the present disclosure relates to a method for processing a biological target material from a liquid sample. The wells of a multiwell plate are inserted into corresponding receptacles of a heating adapter, whereby the sample and a suspension of magnetic particles with a binding surface are added before, during or after. The wells are heated to a predefined temperature, and the biological material is bound to the surface of the magnetic particles. Magnets of a magnetic separation plate are inserted into corresponding recesses in the lower face of the heating adapter, and due to the relative proximity of the receptacles to the recesses, the inserted magnets can impose a magnetic field gradient on the wells and cause the formation of a pellet of the magnetic particles. The supernatant is then withdrawn while the magnetic particles are retained by the magnetic force exerted by the magnets of the magnetic separation plate.

### Short description of the figures

**Fig. 1** is a schematic cross-sectional drawing of the system described herein.
**Fig. 2** depicts the heating device (100) disclosed herein hovering above the magnetic separation plate (310) described herein in a perspective cross-sectional view from above.
**Fig. 3** features a perspective view of a cross-sectioned, fully assembled system described herein, including multiwell plate (200), heating device (100), and magnetic separation device (300).
**Fig. 4** provides a perspective view of a separate heating device (100) seen from above.
**Fig. 5** shows the heating device (100) of Fig. 4 in a perspective view from below.

### Detailed description

The invention provides a system for processing a biological target material from a liquid sample, the system comprising:
- a multiwell plate having a plurality of wells with an open top and a closed bottom, wherein at least a part of the plurality of wells comprises a suspension of magnetic particles with a binding surface;
- a magnetic separation device comprising a magnetic separation plate having a plurality of magnets in a predetermined geometrical arrangement, the magnetic separation device further comprising an actuator for moving the magnetic separation plate relative to the multiwell plate;
- a heating device comprising an essentially non-magnetic heating adapter having an upper face, a lower face and a plurality of receptacles opening towards the upper face, the receptacles being shaped to receive the wells of the multiwell plate, and a heating element between the receptacles on the upper face of the heating adapter, the heating adapter further comprising recesses opening towards the lower face, in positions corresponding to the predetermined geometrical arrangement of the plurality of magnets of the magnetic separation plate, wherein the receptacles and the recesses are in sufficient proximity to allow the inserted magnets to impose a magnetic field gradient onto the corresponding inserted wells.

This approach confers a number of advantages as compared to previous alternatives used in the art.

There is, for example, no need to separate magnetic processing and thermal incubation, as opposed to the teaching of US6368561 B1. The efficient combination of the two processes in terms of both time and space consolidates the isolation of the biological material from the liquid sample in several respects:
The space taken up by the system of the present disclosure can be considerably smaller than if a separate magnetic arrangement and a separate heating plate, not interacting with each other, have to be kept next to each other. Space is a limited resource in many laboratories, especially but not only in medical point-of-care environments. The system disclosed herein is in some embodiments a pre-analytical system. The aim of a pre-analytical system as such is to deliver a sample that can be readily analyzed, which means that one or more analytical systems are used for the respective downstream analysis. In some embodiments of the present disclosure, the system forms an integral part of an analytical system. Such a combined system benefits from direct interaction of the pre-analytical and analytical modules, for instance, within the same housing, thus further reducing the required time for processing and analysis as well as the risk of contamination.

Also, the temporal aspect is improved, as the magnetic field gradient can act on the wells while - at the same time - the thermal incubation can take place. The additional step of translocating the multiwell plate from magnets to heater and back can be omitted, thus accelerating the overall process and creating the possibility to increase sample throughput. Decreasing time-to-result can be crucial, especially in clinical settings where diagnostic results often have to be obtained within the shortest time possible in order to initiate the appropriate medical treatment.

Furthermore, in the case of an approach with separate heating and magnetic attraction, the liquid sample has to leave the heating device in order to interact with the magnets, resulting in the risk of temperature decrease within that period, which might adversely affect the isolation procedure.

The system disclosed herein, by contrast, allows for uninterrupted thermal incubation, while the magnets impose their magnetic field gradients onto the wells of the multiwell plate.

The structure of the essentially non-magnetic heating adapter disclosed herein, with the receptacles for the wells and the recesses for the magnets in relatively close proximity to each other, is favored by the heating element being applied to the upper surface of the heating adapter.

### Terms

"Biological target material" or "biological material", in the sense of this disclosure, comprises all kinds of biological molecules, for example proteins or nucleic acids, but also other molecules occurring in nature or being derivatives or synthetic analogues or variants thereof. Furthermore, the term "biological material" comprises viruses and eukaryotic and prokaryotic cells. In some embodiments, the biological target material is nucleic acids such as e.g. DNA, RNA or PNA. The DNA can be e.g. viral DNA, genomic DNA or plasmid DNA. The biological target material can be native or modified. Native biological material is not irreversibly altered as compared to the respective naturally occurring biological material, such as e.g. DNA or RNA isolated from organisms. Modified biological material comprises e.g. biotinylated molecules such as nucleic acids or proteins.

As used herein, the term "liquid sample" refers to a liquid material that may potentially contain an analyte of interest. In embodiments where the "liquid sample" is a "liquid biological sample", the sample can be derived from any biological source, such as a physiological fluid, including blood, saliva, ocular lens fluid, cerebrospinal fluid, sweat, urine, stool, semen, vaginal fluid, milk, ascites fluid, mucous, synovial fluid, peritoneal fluid, amniotic fluid, tissue, cultured cells, or the like. The test sample can be pretreated prior to use, such as preparing plasma from blood, diluting viscous fluids or diluting in general, lysis or the like. Methods of treatment can involve filtration, distillation, concentration, inactivation of interfering components, and the addition of reagents. A biological sample may be used directly as obtained from the source or used following a pretreatment to modify the character of the sample. In some embodiments, an initially solid or semi-solid biological material is rendered liquid by dissolving or suspending it with a suitable liquid medium. In further embodiments, the sample may have been pre-treated with, for example, a washing buffer. In some embodiments, the biological sample is suspected to contain a certain antigen or nucleic acid.

The "magnetic particles" disclosed herein may be particulate material such as beads including nanobeads, or the like. In some embodiments, they are analyte binding particles for binding specific biological targets which may, for example, be molecules, cells or viruses. In those embodiments, the particles may have surfaces coated with specific or unspecific binding molecules, such as nucleic acid capture probes, oligo- or poly(dT)-strands for binding mRNA, protein A for binding the Fc parts of immunoglobulins, Fab fragments of antibodies for binding specific proteins, nickel for binding histidine tags, streptavidin or biotin, integrins, adhesins, or other cell-surface molecules, or the like. In some embodiments, the biological target molecules are cell surface molecules, such that specific cells may be captured by the analyte binding particles. For example, for blood samples, suitable antibodies specifically binding to cell surface antigens of leucocytes, erythrocytes, monocytes are known to people skilled in the art, for example, CD2/CD3 for T cells, CD14 for monocytes, CD15 for granulocytes and monocytes, CD16 for macrophages, CD36 for platelets, monocytes and macrophages, CD45 for leucocytes. In further embodiments, the analyte binding particles have a metal-oxide or silica surface. Silicon dioxide surfaces such as glass surfaces may be used to bind nucleic acids in the presence of chaotropic agents. Magnetic particles especially useful in the context disclosed herein include the ones taught in EP 2916327 (nanobeads) or EP 1281714. As described above, nanobeads such as the ones disclosed in EP 2916327 are of special advantage due to their physical properties, including the relatively low sedimentation coefficient resulting in a low sedimentation speed. While the methods and devices disclosed herein can be carried out with paramagnetic, superparamagnetic, ferro- or ferrimagnetic particles, all of which are parts of embodiments of the present disclosure, the particles of EP 2916327 are ferrimagnetic. More precisely, they are monodisperse silanized ferrimagnetic iron oxide particles for independent generic nucleic acid binding, the particles having the following characteristics: A core comprising an inner layer comprising Fe₃O₄ and an outer layer comprising Fe₂O₃, a coating, wherein the coating comprises silica and silicates from sodium silicate precipitation, a sedimentation speed in pure water of less than 60 µm/s, and no significant iron bleeding occurs on the particles in 1M HCl for at least 60 min. In some embodiments, the magnetic particles have a mean diameter of 50 nm to 50 µm, or of 100 nm to 25 µm, or of 500 nm to 5 µm. In a specific embodiment, the magnetic particles have a mean diameter of about 1 µm.

In one embodiment, the difference in size of monodisperse silanized ferrimagnetic iron oxide particles is in average smaller than 5%. In a specific embodiment, the size or diameter of the particles has a value of n, wherein n is a value from 20 nm to 600 nm. In a more specific embodiment, the size or diameter of the particles has a value of n, wherein n is 100 nm.

In the context described herein, a "multiwell plate" constitutes an essentially flat plate comprising a multitude of reaction chambers in the form of wells or cavities which are used as test tubes for samples to be subjected to biological or chemical assays, wherein the multiwell plate can be made from any suitable kind of available material, such as glass, plastics, quartz, or silicon and typically provides 6, 24, 48, 96, 384, 1536 or even more sample wells which are often arranged in an mxn pattern (with m and n being positive integers), for instance, a 2x3 rectangular matrix. In cases where the multiwell plate complies with ANSI/SLAS (previously known as SBS) standards, they may be directly used in or with equally standardized devices and systems, such as multipipettors, magnetic plates, optical analyzers, and the like. The wells of a multiwell plate may be chemically inert on the inside, such that they do not interfere with the analytical reactions taking place within. In other embodiments, they may be coated with binding molecules such as biomolecules. Examples for biomolecules that may act, for instance, as capture molecules for binding either a target nucleic acid or other nucleic acids, include sequence-specific nucleic acid capture probes, such as DNA or LNA (Locked Nucleic Acid) probes. Another example would be streptavidin for interaction with a biotin tag at the target nucleic acid. Multiwell plates useful in the context described herein may have wells with diameters or wrench sizes - measured at the well opening which may, for example, be round, polygonal such as hexagonal, or the like - in the milli- to centimeter range, for example from 1 mm to 5 cm, or from 2.5 mm to 2.5 cm, or from 5 mm to 1.5 cm, or any combination of these ranges. In some embodiments, the wells have a diameter or wrench size of about 1 cm. In the frequently used m x n arrangement, with m denoting a vertical and n denoting a horizontal position, the distance or spacing between two wells in an m- or n-position may be between 4.5 and 18 mm, or between 7 and 12 mm, or about 9 mm. In the case of standard SBS 96-well plates, the distance in both m- and n-direction is 9 mm. In other embodiments, it may be the case that the distance in the m- and the distance in the n-direction are different from each other.

Multiwell plates as described herein may comprise their wells in an optically transparent area. Suitable materials conferring high optical transparency and a low level of autofluorescence include, for instance, glass, plastics, quartz, silicon, or the like. In some embodiments, the material is a cyclic olefin polymer (COP) or copolymer (COC). Other suitable materials are known to the person skilled in the art. In some embodiments, the entire multiwell plate is made of the same optically transparent material. In other embodiments, a non-transparent area, for example, towards the edges of the multiwell plate, may be made of a different material such as a more robust material for handling and protection purposes or the like.

In some embodiments described herein, the multiwell plate has dimensions according to the standard SBS format, i.e., in compliance with ANSI/SLAS standards. Such standardized dimension may be very advantageous with regard to usability of commercially available components. For instance, in some embodiments, the multiwell plate itself may be a commercially available multiwell plate as found in most laboratories as a part of the standard equipment. Manufacturers include Bio-Rad, Greiner, and others.

For the purposes of the methods and systems disclosed herein, the multiwell plate comprises recesses in positions corresponding to a predetermined geometrical arrangement of a plurality of magnets. Advantageously, the recesses are formed on the face of the plate opposite from the wells. For instance, a recess may be located in the center of four wells arranged in an mxn pattern, but on the opposing side. Thus, the magnet - especially in embodiments where the magnet has a cuboidal, rod- or pin-like shape - can be introduced into the recess and thereby positioned in close proximity of the four wells surrounding it, hence imposing a magnetic gradient on the wells and their contents.

A "magnetic separation plate" is a device useful for the separation of magnetic particles. It comprises a "support plate" and magnets, wherein the "support plate" is typically an essentially flat device for bearing and holding the magnets in a defined position which is usually perpendicular to the support plate. Said plate can be made of one or more parts and different materials such as metal or plastic. In an embodiment, the plate is made of metal. The support plate may comprise an upper and a lower plate fastened to each other.

In some embodiments described herein, the magnetic separation device further comprises on its upper face a frame for receiving the heating device and the multiwell plate. Such structures may facilitate alignment between the distinct components of the system disclosed herein.

In some embodiments, the magnets are essentially pin- or rod-shaped magnetic or magnetizable structures. The length of such magnetic pins or rods can be 2-100 mm, or 15-50 mm. Their diameter can be 1-20 mm, or 2-6 mm. In further embodiments, the magnets are essentially cuboidal. In more specific embodiments, these cuboids stretch along the x- or y-axis of a magnetic separation plate with dimensions corresponding to a multiwell plate having an SBS format. Correspondingly, such cuboids may have a length of about 7.5 cm (in y-direction), or about 12 cm (in x-direction). The height may be between about 5 mm to 10 mm. In embodiments where the multiwell plate is a standard SBS 96-well plate, as shown in the Figures, then the width of a cuboidal magnet may be between 2 and 3 mm, such that they fit into the recesses between the wells.

In the absence of external forces acting on the magnets, the angle between the magnets and the support plate can be essentially a right angle. In an embodiment, the angle is between 80 and 100°, or between 85° and 95°, or about 90°.

A "predetermined geometrical arrangement" in the context of the present disclosure means a defined spatial and directional relation between elements of a group of physical objects such as magnets or corresponding recesses, or receptacles and wells.

A "heating device", in the context of the present disclosure, comprises an essentially non-magnetic "heating adapter" having an upper face and a lower face. The upper face contains receptacles in which the wells of the multiwell plate may be inserted such that they fit snugly. Thus, the shape of the receptacles may essentially correspond to the outer shape of the wells, such that a substantial percentage of the surface of the outer well wall is in thermal contact with the receptacle of the heating adapter. "Thermal contact" means either direct physical contact or a contact mediated by a thermally conductive medium, such that an essentially unobstructed heat transfer between heating adapter and wells is possible. The lower face contains recesses in a geometrical arrangement matching the arrangement of the magnets of the magnetic separation plate, while the geometrical arrangement of the receptacles on the upper face matches the geometrical arrangement of the wells of the multiwell plate. As a result, receptacles and recesses are in sufficiently close proximity to each other to enable the inserted magnets to magnetically interact with the inserted wells. Suitable shapes of the "heating adapter" include, for instance, crisscrossed or comb-like structures, cuboidal structures, ashlar-shaped structures, honey-comb structures, trapezoidal structures, or other suitable geometries. The heating adapter may be made from materials like, for example, aluminum and its alloys, copper, steel, silver, alumina ceramics, and/or silicon carbide.

In embodiments where the essentially non-magnetic heating adapter is made from metal, a further advantage is achieved by facilitating liquid level detection by means of, for example, capacitive liquid level detection methods, as commonly used in automated liquid handling systems.

The distance between receptacles and recesses, and hence between the magnets and the wells when the wells and the magnets are fully inserted, is in some embodiments relatively small in order to exert the maximum magnetic force to the beads in the wells when the magnets are in position to collect the magnetic particles. In some embodiments, the distance between a magnet and the well can be from about 0.2 mm, 0.5 mm, or 1 mm to about 2.5 mm, 5 mm, or 1 cm. In some embodiments, the distance is about 0.2 mm. The design is adapted to reduce the distance between the magnets and the magnetic particles in the wells in order to have the greatest magnetic field gradients, resulting in the largest magnetic force and separation speed of the beads. At the same time the manufacturability has to be considered.

In some embodiments the receptacles may not fully embrace the wells in order to approximate the magnets even closer to the well. In such a case the well is not fully surrounded by the thermally conductive material of the heating adapter, but for instance includes a slit through which the magnet can be inserted and thus brought into direct or almost direct physical contact with the corresponding well. In such embodiments, the well will still be embraced to a significant extent by the thermally conductive material of the heating adapter in order to ensure sufficient transfer of thermal energy. The slit(s) in the heating adapter are therefore in some embodiments only as large as required to permit the respective magnet access to the corresponding well.

In some embodiments of the present disclosure, the system disclosed herein further comprises one or more elements selected from the group of a pipettor, a control unit, and a data management unit.

A "pipettor" is a device allowing for the automatic withdrawing and/or dispensing of volumes of fluids such as for fluid transfer or sip and spit mixing. In the context described herein, these fluids include the liquid biological sample, reagents used for processing the liquid biological sample, cleaning solutions, dilution buffers, processed liquids, liquids containing a processed analyte, or the like. The liquids may be withdrawn and dispensed from any of the following positions/vessels: sample tubes, intermediate process tubes, reagent containers, waste containers or positions, tip-wash-stations, output vessels, reaction tubes, and the like. In particular, the pipettor may be used for the dispensing of a fluid biological sample into the wells of the multiwell plate described herein, or withdrawing it therefrom. The pipettor is in some embodiments driven by a pneumatic or hydraulic system. As a hydraulic liquid, the pipettor may in some embodiments use water or a commonly used reagent as known to the person of skill in the art.

The pipettor may comprise one or more reusable washable needles such as a steel needle, or use disposable pipette tips. The pipettor may be mounted to a transfer head that can be moved in one or two directions of travel in a plane, for example, with guiding rails and a third direction of travel orthogonal to the plane, with a spindle drive or the like. For instance, the pipettor may be moved horizontally between a primary sample tube and the multiwell plate or another target position, and vertically in order to withdraw or dispense the liquid biological sample or other liquids. The pipettor may be integrated, i.e. built in a work cell or be a module of a system operatively connected to a work cell. The position and operation (including parameters such as volume, flow rate, direction of flow, or the like) of the pipettor are controlled by a control unit, as described herein.

A "control unit" controls an automated system in a way that the necessary steps for the processing protocols are conducted by the automated system. That means the control unit may, for example, instruct the automated system to conduct certain pipetting steps with a pipettor to mix the liquid biological sample with reagents, or the control unit controls the automated system to incubate the biological sample or reagents or mixtures of both for a certain time at a certain temperature, or the control unit controls the precise movements of the magnetic separation plate, and/or the multiwell plate, and/or the pipettor described herein, or other movements or related parameters. The control unit may receive information from a data management unit (DMU) regarding which steps need to be performed with a certain sample. In some embodiments, the control unit might be integral with the data management unit or may be embodied by a common hardware. The control unit may, for instance, be embodied as a programmable logic controller running a computer-readable program provided with instructions to perform operations in accordance with a process operation plan. In particular, the control unit may include a scheduler, for executing a sequence of steps such as the movements described above within a predefined time. The control unit may further determine the order of samples to be processed according to the assay type, urgency, and the like. The control unit may also receive data from a detection unit related to a measurement of parameter of the sample.

A "data management unit" is a computing unit for storing and managing data. This may involve data relating to the liquid sample to be processed by the automated system, or data relating to the steps to be carried out within the rotatable vessel. The data management unit may be connected to an LIS (laboratory information system) and/or an HIS (hospital information system). The data management unit (DMU) can be a unit within or co-located with the automated system it interacts with. It may be part of the control unit. Alternatively, the DMU may be a unit remotely located from the automated system. For instance, it may be embodied in a computer connected via a network to the automated system.

In some embodiments of the system disclosed herein, the heating device further comprises a temperature sensor. It may be desirable to monitor the temperature of the heating device or the essentially non-magnetic heating adapter, respectively. In some instances, processing of the liquid sample may require incubation at a specific temperature, the correctness of which may be surveyed by the temperature sensor. In the case, for example, of deviation beyond a pre-defined threshold, it may be advantageous to adjust the temperature of the heating device or the heating adapter specifically to the desired value or range by adjusting the power supplied to the heating element. Such controlled supply may be steered by the control unit or a part thereof. The temperature of the heating device or particularly the heating adapter may be controlled by a temperature controller comparing an effective temperature with a target temperature. The controller may, for instance, use a PID (Proportional-Integral-Derivative) algorithm to reduce the deviation between effective and target temperature, as known to the skilled artisan.

The heating element can, for instance, be realized by applying an electrical resistive heating element to the upper face of the heating adapter. This may be accomplished by applying a suitably shaped heating element to the surface of the heating adapter, using, for example, a thermally resistant adhesive. Such a heating element may be made from a carrier material, such as from Polyimide (Kapton®) or silicon-polymer, including an electrically conductive material, generating heat under electrical current. The heating element may include a thermal sensor. The production and application of such adhesive heating elements is known to the person of skill in the art.

Alternatively, an electrically conductive, resistive heater material may be printed to the upper face of the heating adapter using so called "thick-film"-technology. In order to avoid an electrical shortcut, the heating adapter, especially if electrically conductive, is in some embodiments coated with an electrical isolation layer. Such an electrical isolation layer may be, for instance, made of an oxide such as aluminum oxide. Screen printing may be used to apply the resistive heater material to the heating adapter. Such printable resistive heater materials typically include an electrically conductive powder, such as silver, copper or carbon and a binder such as a curable epoxy resin. The production and application of such thick-film heaters is also known to the skilled person.

Furthermore, an electrically conductive resistive heater material may be sputtered or vacuum-deposited onto the heating adapter using so called "thin-film"-technology. Suitable materials for this purpose include gold or platinum. As in thick-film techniques, an electrical isolation layer may be applied to avoid electrical shortcuts. The shape of the heater may be defined, for instance, by a mask process or by laser ablation. The production and application of such thin film heaters is well-established in the art.

As a further option, electrical resistive wires may be applied to the heating adapter. Such wires may, for example, be made of constantan. Alternatively, the heating adapter may have one or more fluid paths to transport thermal transport media.

"Thermal transport media", as understood in the context of the present disclosure, mean fluids of sufficient thermal conductivity to transfer thermal energy throughout the heating element of the heating adapter. Suitable fluids are known to the person skilled in the art.

A further aspect disclosed herein is a method for processing a biological target material from a liquid sample, the method comprising the steps of:
a) providing a multiwell plate having a plurality of wells with an open top and a closed bottom;
b) inserting the wells of the multiwell plate into corresponding receptacles on the upper face of an essentially non-magnetic heating adapter comprised by a heating device, the receptacles being shaped to receive the wells of the multiwell plate;
c) adding the liquid sample and a suspension of magnetic particles with a binding surface either individually or together to at least a part of the plurality of wells before, during, or after any of the steps a) or b);
d) heating the liquid sample in the wells with a heating element between the receptacles on the upper face of the heating adapter to a predefined temperature and incubating the liquid sample and the magnetic particles, using conditions under which the biological target material binds to the surface of the magnetic particles;
e) introducing a plurality of magnets of a magnetic separation plate into corresponding recesses in a lower face of the heating adapter, wherein each magnet is brought into sufficient proximity to the outside wall of a corresponding well to impose a magnetic field gradient onto the inner space of said well through the heating adapter, thereby forming a pellet of magnetic particles;
f) withdrawing the liquid from the respective wells with a plurality of pipette tips of a pipettor while retaining the pellet of magnetic particles by magnetic force.

The method described herein may especially advantageously be carried out with the system described herein, including any specific embodiments disclosed in the context of the system described herein. Hence, the advantages of the system described herein also apply to the method described herein.

In some embodiments, the method described herein further comprises the following steps:
g) adding an elution buffer to the wells and resuspending the magnetic particles therein;
h) eluting the biological target material from the magnetic particles with the elution buffer.

An "elution buffer" is a suitable liquid for separating biological target material from a solid support to which it is bound, such as the magnetic particles described herein. Such a liquid may, for instance, be distilled or deionized water or aqueous salt solutions such as Tris buffers like Tris HCl, or HEPES, or other suitable buffers known to the skilled artisan. The pH value of such an elution buffer is preferably alkaline or neutral if the biological target material is a nucleic acid. Said elution buffer may contain further components such as preserving agents like chelators like EDTA for stabilizing the isolated biological target material such as nucleic acids by inactivation of degrading enzymes. In some embodiments, elution buffers like nucleic acid elution buffers have a low salt concentration, such as 0.1 M or lower. In many cases, the elution process as such may also be temperature-dependent, or can at least be influenced by varying the temperature. Therefore, in some embodiments, the thermally controlled conditions provided by the system or through the method described herein may also be applied on the elution step(s).

Therefore, in some embodiments, elution of the biological material from the binding surface of the magnetic particles involves heating the elution buffer in the wells with the heating element between the receptacles on the upper face of the heating adapter to a predefined temperature and incubating the elution buffer and the magnetic particles, using conditions under which the biological target material is eluted from the surface of the magnetic particles.

Likewise, any potential washing step may be subject to thermal control using the system and/or method described herein. A washing step may be performed at any stage of the method described herein as long as the biological material is bound to the surface of the magnetic particles. One appropriate stage is, for example, after step f) if the method described herein. The conditions for such a washing step are such that the material remains bound and is not eluted. This step typically includes a washing buffer.

A "washing buffer" is a liquid designed to remove undesired components, especially in a purification procedure. Such buffers are well known in the art. The washing buffer is suited to wash the magnetic particles in order to separate the immobilized analyte from any unwanted components. Often the washing buffer or other solutions are provided as stock solutions which have to be diluted before use. The washing buffer may, for example, contain ethanol and/or chaotropic agents in a buffered solution or solutions with an acidic pH without ethanol and/or chaotropic agents.

In such embodiments, the method described herein further comprises a step of adding a washing buffer to the wells for removing undesired components from the surface of the magnetic particles while retaining the biological target material thereon, then withdrawing the liquid from the respective wells while retaining the pellet of magnetic particles by magnetic force.

As in the case of the elution step, in some embodiments, washing the magnetic particles with the biological material bound to their surface involves heating the suspension in the wells with the heating element between the receptacles on the upper face of the heating adapter to a predefined temperature and incubating the suspension, using conditions under which the biological target material remains bound to the surface of the magnetic particles.

The method disclosed herein may also be used for washing magnetic particles with biological material bound to it, even if the binding step was performed previously using a different method. In such embodiments, a washing buffer may, for example, be added in step c), while the conditions in step d) serve for keeping the biological material bound to the surface of the magnetic particles, which may in some embodiments be essentially the same conditions as for establishing the binding. Likewise, the system described herein may serve for washing - and/or eluting - the biological material on/from the magnetic binding particles, even if the binding step was performed on a different system.

Further, the thus purified biological material may in some embodiments be subjected to biological or biochemical analysis. Such downstream analysis of the biological target material, as may be performed in a step i) of the method disclosed herein, may include Polymerase Chain Reaction (PCR) or sequencing of nucleic acids, or antibody-mediated assays for proteins such as ELIZA, or the like. In some embodiments of the method described herein, the analysis comprises qualitative and/or quantitative detection of nucleic acids by amplification. In some embodiments, the amplification technique is PCR. Other amplification techniques, such as isothermal amplification (LAMP, TMA, and the like), LCR, etc. may be applied for analyzing a target nucleic acid. In more specific embodiments, the analysis comprises nucleic acid sequencing. Also in some embodiments, the analysis comprises library preparation for nucleic acids.

### Exemplary embodiments

The following Examples are meant to illustrate specific embodiments of the systems and methods of the present disclosure, while they are not limiting.

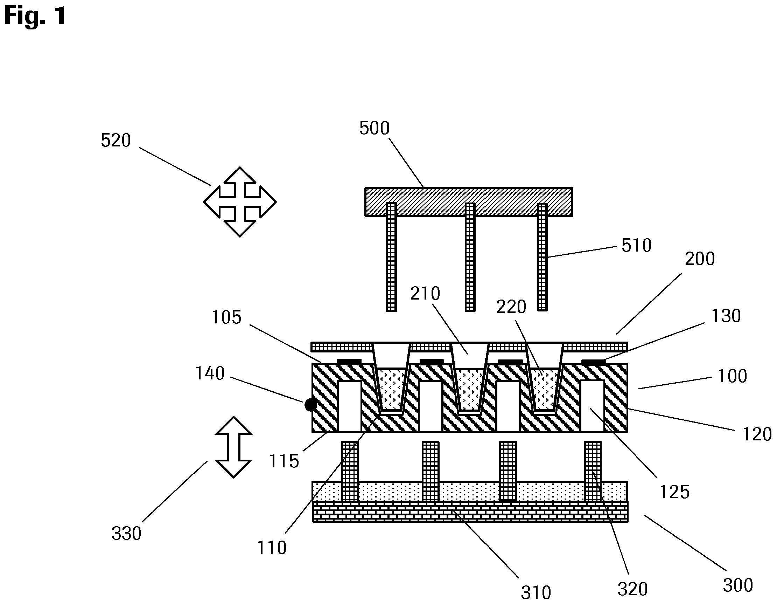

The schematic drawing of **Fig. 1** depicts an embodiment of the system described herein. The cross-sectional side view shows a heating device (100) with the wells (210) of a multiwell plate (200) inserted into the corresponding receptacles (110) opening towards the upper face (105) of the essentially non-magnetic heating adapter (120). A heating element (130) is placed on the upper face (105) of the heating adapter (120), in this embodiment as a multi-part element evenly distributed between the receptacles (110). The application of the heating element (130) on the upper surface (105) of the essentially non-magnetic heating adapter (120) reduces the complexity of the heating adapter (120) as such. Building a heating element into the body of the heating adapter (120) would require space and in most cases result in a thicker heating adapter (120), including the risk of increasing the distance that a magnetic field gradient would have to extend over between magnets (320) and wells (210) in order to apply a sufficiently strong field gradient onto the wells (210) and the liquid sample (220) with the suspension of magnetic particles (230) contained therein. The setup described herein, in contrast, allows for a thin heating adapter (120) with low complexity, in some embodiments consisting essentially of a layer of non-magnetic thermally conductive material and the heating element (130) applied to its upper face (105). The heating device (100) shown in Fig.1 further includes a temperature sensor (140) as a further part of the essentially non-magnetic heating adapter (120). As explained herein, it may be of considerable advantage to be able to monitor the temperature within the heating device (100) or the heating adapter (120), respectively, since many chemical reactions benefit from or even require a specific optimum temperature or temperature range.

The relative magnetic permeability of the essentially non-magnetic heating adapter (120) is in some embodiments < 5 [1], in more specific embodiments < 1.5[1]. The thermal conductivity of the essentially non-magnetic heating adapter (120) is in some embodiments > 20 [Wm⁻¹/K⁻¹], in more specific embodiments > 100 [Wm⁻¹K⁻¹]. As mentioned above, one of the suitable materials for the essentially non-magnetic heating adapter (120) is aluminum, including its alloys.

For the sake of interaction with the magnets (320) mentioned above, the essentially non-magnetic heating adapter (120) further possesses recesses (125) in its lower face (115), in positions corresponding to the predetermined geometrical arrangement of the plurality of magnets (320) of the magnetic separation plate (310) depicted below the heating adapter (120) as part of a magnetic separation device (300). The double-headed vertical arrow represents an actuator (330) for moving the magnetic separation plate (310) relative to the multiwell plate (200), in this embodiment along a vertical axis. As can be deduced from the schematic positioning of the magnets (320) in spatial relation to the recesses (125), the actuator (330) is arranged to vertically insert the magnets (320) into the recesses (125) or retract them therefrom, depending on the respective step of the method described herein.

Positioned above the multiwell plate (200) mounted on the heating device (100) is a fluid handling system, in this embodiment a pipettor (500) with multiple pipetting needles or pipette tips (510). The four-headed arrow represents an actuator (520) moving along vertical and horizontal axes in order to move the pipettor (500) and any collected liquid vertically towards and away from the wells (210) of the multiwell plate (200) and horizontally to other modules or parts of, for example, an analyzer or a pre-analytical apparatus. For instance, the pipettor (500) may pick up reagent from a remote reagent container (not shown) by vertical motion and, subsequently, horizontally transport it to the multiwell plate (200) before resuming a vertical movement for the pipetting procedure.

Turning to the method described herein, Figure 1 depicts a step in which the wells (210) of the multiwell plate (200) have been inserted into the corresponding receptacles (110) opening towards the upper face (105) of the essentially non-magnetic heating adapter (120) of the heating device (100).

In the following, the liquid sample (220) in the wells (210) is heated with the help of the heating element (130) to a predefined temperature, using conditions under which a biological target material, if present in the liquid sample (220), binds to the surface of the magnetic particles (230). The material in question may thereby be separated from the non-desired biological material present in the liquid sample (220), depending on the application and the particles involved. For instance, specific nucleic acids may be bound to oligonucleotides immobilized on the surface of the magnetic particles (230). Such oligonucleotides may act as capture probes specifically binding nucleic acids of a certain sequence. In other cases, nucleic acids may be bound non-specifically to a glass or silica surface of the magnetic particles (230) under chaotropic conditions. Once the target material has been bound, the magnets (320) of the magnetic separation plate (300) are inserted into the corresponding recesses (125) in the lower face (115) of the heating adapter (120), wherein each magnet (320) is brought into sufficient proximity to the outside wall of a corresponding well (210) to impose a magnetic field gradient on the inner space of said well (210) through the heating adapter (120). The application of magnetic force leads to the formation of a pellet (240) of magnetic particles (230), such that they are held back at the inner wall of the vessel (210). In the following, the surrounding liquid from the sample (220) is aspirated through the pipette tips (510) of the pipettor (500) without disturbing the pellet (240). If required, another liquid may subsequently be added, such as an elution buffer, a reaction mixture, or the like. As described herein above, resuspending the particles (230) in an elution buffer under appropriate conditions, as known by the person skilled in the art, leads to the liberation of the biological target material from its attachment to the surface of the magnetic binding particles (230). The eluted and, in many cases, purified material may then be processed further. For instance, it may be withdrawn from the vessel (210), dissolved in the elution buffer, with the help of the pipette tips (510) of the pipettor (500), while the magnetic particles (230) may be held back by magnetic force as described before. A wide range of analytic tools and techniques is available to the person skilled in the art to be applied upon the purified biological target material and includes, for instance, nucleic acid sequencing or amplification methods.

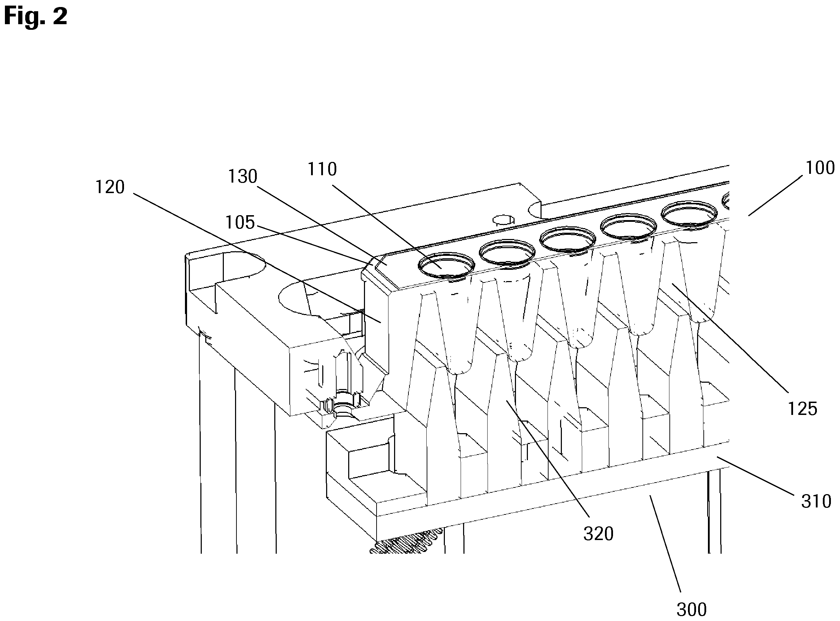

The schematic drawing of **Figure 2** provides a perspective cross-sectional view of a specific part of the system described herein. Part of the outermost row of the heating device (100) with the essentially non-magnetic heating adapter (120) can be seen, including the plurality of receptacles (110) opening towards the heating adapter's (120) upper face (105). The heating element (130) in this embodiment forms a layer on top of the upper face (105). The magnets (320) of the magnetic separation device (300) in this depiction are only partially introduced into the corresponding recesses (125) in the lower face (115) of the essentially non-magnetic heating adapter (120). However, it can be appreciated that the geometrical shape of magnets (320) and corresponding recesses (125) facilitate a snug fit between these elements when engaged.

The system in Figure 2 is displayed without the multiwell plate (200) for the sake of clarity. However, it can be gathered how a standard SBS multiwell plate may be mounted onto the heating adapter (120) by placing the wells (210) into the corresponding receptacles (110). The outer rim of the multiwell plate (200) would then cover the upper face (105) and the circumference of the heating adapter (120) in a skirting-like manner.

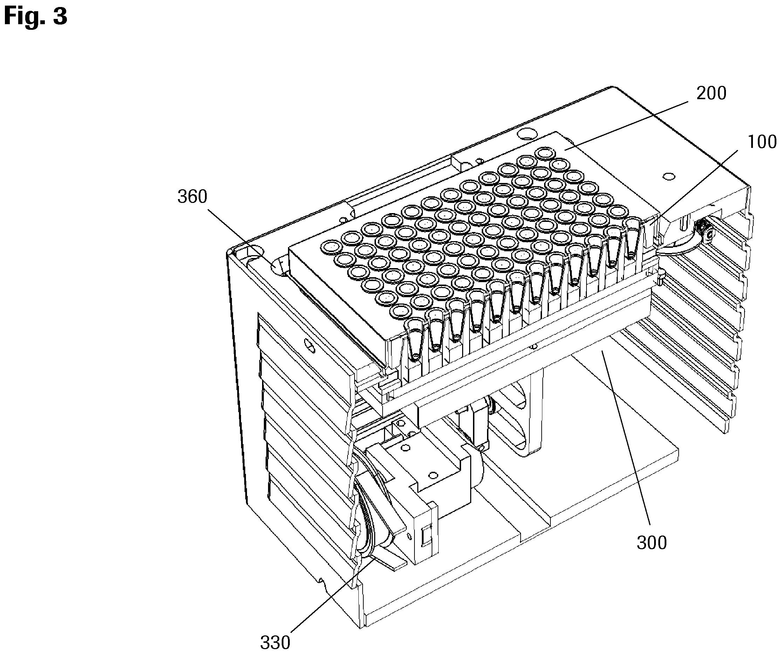

The latter can be observed in **Figure 3****,** in which the multiwell plate (200) is placed upon the heating device (100) with the essentially non-magnetic heating adapter (120). The magnets (320) of the magnetic separation device (300) in this depiction have been fully inserted into the corresponding recesses (125) in the lower face (115) of the essentially non-magnetic heating adapter (120). With regard to the method described herein, such positioning would facilitate the formation of a pellet (240) of magnetic particles (230) within the wells (210) of the multiwell plate (200). The distance between magnets (320) and wells (210), separated only by the essentially non-magnetic heating adapter (120), is sufficiently small to allow the magnets (320) to impose a magnetic field gradient onto the wells (210) including the magnetic particles (230) in the fluid samples (220) contained therein.

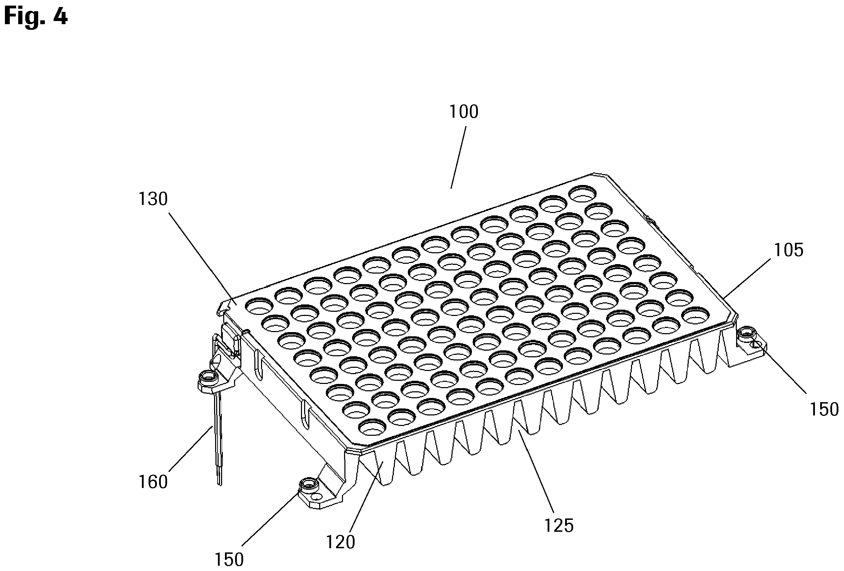

**Figure 4** provides a perspective view of an isolated heating device (100) as described herein. In this depiction, seen from above, the upper face (105) of the essentially non-magnetic heating adapter (120) with its plurality of receptacles (110) for receiving the wells (210) of a multiwell plate (200) is visible. The heating element (130) forms a layer placed on top of the upper face upper face (105) of the essentially non-magnetic heating adapter (120) and, in this embodiment, thereby mostly covers it. The depicted embodiment features electrical leads (160) for supplying the heating adapter (120) or its heating element (130), respectively. Further visible in this depiction is a mounting structure (150) for fixing the heating device (100) to a frame. Such a frame may be part of a housing comprised by the magnetic separation device (300), as shown in Figure 3.

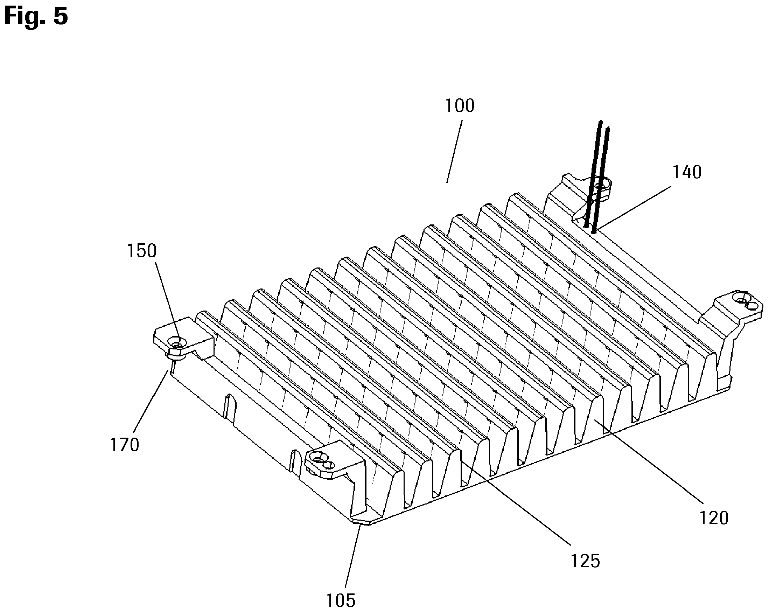

The lower face (115) of the essentially non-magnetic heating adapter (120) of Figure 4 is displayed in **Figure 5****,** providing a perspective view of the heating device (100) from below. The recesses (125) for receiving the magnets (320) of the magnetic separation plate (310) comprised by the magnetic separation device (300) are clearly visible in this depiction. In line with the elongated shape of the recesses (125), arranged for snugly fitting corresponding magnets (320) with a cuboidal shape as shown in Figure 3, the essentially non-magnetic heating adapter (120) exhibits a crisscrossed, comb-like geometrical structure. Further visible is the temperature sensor (140) described before, as well as the mounting (150) that, in this embodiment, comprises a thermally isolating material (170), such as a ferrule made of thermally nonconductive material, for instance, PEEK or a thermally nonconductive ceramic.

In general, modifications and variations of all disclosed embodiments are possible in light of the above description. It is therefore to be understood, that within the scope of the appended claims, the invention may be practiced otherwise than as specifically devised in the above examples. Reference throughout the preceding specification to "one embodiment", "an embodiment", "one example" or "an example", means that a particular feature, structure or characteristic described in connection with the embodiment or example is included in at least one embodiment. Thus, appearances of the phrases "in one embodiment", "in an embodiment", "one example" or "an example", in various places throughout this specification are not necessarily all referring to the same embodiment or example.

Furthermore, the particular features, structures, or characteristics may be combined in any suitable combinations and / or sub-combinations in one or more embodiments or examples The scope of the invention is defined by the appended claims.

## Claims

1. A system for processing a biological target material in a liquid sample (220), the system comprising:
- a multiwell plate (200) having a plurality of wells (210) with an open top and a closed bottom, wherein at least a part of the plurality of wells (210) comprises a suspension of magnetic particles (230) with a binding surface;
- a magnetic separation device (300) comprising a magnetic separation plate (310) having a plurality of magnets (320) in a predetermined geometrical arrangement, the magnetic separation device (300) further comprising an actuator (330) for moving the magnetic separation plate (310) relative to the multiwell plate (200);
- a heating device (100) comprising an essentially non-magnetic heating adapter (120) having an upper face (105), a lower face (115) and a plurality of receptacles (110) opening towards the upper face (105), the receptacles (110) being shaped to receive the wells (210) of the multiwell plate (200), and a heating element (130) between the receptacles (110) on the upper face (105) of the heating adapter (120), the heating adapter (120) further comprising recesses (125) opening towards the lower face (115), in positions corresponding to the predetermined geometrical arrangement of the plurality of magnets (320) of the magnetic separation plate (310), wherein the receptacles (110) and the recesses (125) are in sufficient proximity to allow the inserted magnets (320) to impose a magnetic field gradient onto the corresponding inserted wells (210).

2. The system of claim 1, further comprising one or more elements selected from the group of a pipettor (500), a control unit, and a data management unit.

3. The system of any of the preceding claims, wherein the heating device (100) further comprises a temperature sensor (140).

4. The system of any of the preceding claims, wherein the heating element (130) comprises one or more elements selected from the group consisting of an electrically conductive resistive material and a fluidic or microfluidic system containing thermal transport media.

5. The system of any of the preceding claims, wherein the heating element (130) is printed onto the upper surface of the heating adapter (120).

6. The system of any of the preceding claims, wherein the heating element (130) is sputtered or vacuum-deposited onto the upper face (105) of the heating adapter (120).

7. The system of any of the preceding claims, wherein the multiwell plate (200) has dimensions according to the standard SBS format.

8. The system of any of the preceding claims, wherein the magnetic separation device (300) further comprises on its upper face a frame (360) for receiving the heating device (100) and the multiwell plate (200).

9. The system of any of the preceding claims, wherein the heating adapter (120) is made of one or more materials selected from the group consisting of aluminum and its alloys, copper, steel, silver, alumina ceramics, and silicon carbide.

10. A method for processing a biological target material in a liquid sample (220), the method comprising the steps of:
a) providing a multiwell plate (200) having a plurality of wells (210) with an open top and a closed bottom;
b) inserting the wells (210) of the multiwell plate (200) into corresponding receptacles (110) on the upper face (105) of an essentially non-magnetic heating adapter (120) comprised by a heating device (100), the receptacles (110) being shaped to receive the wells (210) of the multiwell plate (200);
c) adding the liquid sample (220) and a suspension of magnetic particles (230) with a binding surface either individually or together to at least a part of the plurality of wells (210) during, or after any of the steps a) or b);
d) heating the liquid sample (220) in the wells (210) with a heating element (130) between the receptacles (110) on the upper face (105) of the heating adapter (120) to a predefined temperature and incubating the liquid sample (220) and the magnetic particles (230), using conditions under which the biological target material binds to the surface of the magnetic particles (230);
e) introducing a plurality of magnets (320) of a magnetic separation plate (300) into corresponding recesses (125) in a lower face (115) of the heating adapter (120), wherein each magnet (410) is brought into sufficient proximity to the outside wall of a corresponding well (210) to impose a magnetic field gradient onto the inner space of said well (210) through the heating adapter (120), thereby forming a pellet (240) of magnetic particles (230);
f) withdrawing the liquid from the respective wells (210) with a plurality of pipette tips (510) of a pipettor (500) while retaining the pellet (240) of magnetic particles (230) by magnetic force.

11. The method of claim 10, further comprising the following steps:
g) adding an elution buffer to the wells (210) and resuspending the magnetic particles (230) therein;
h) eluting the biological target material from the magnetic particles (230) with the elution buffer.

12. The method of claim 11, wherein elution of the biological target material from the binding surface of the magnetic particles (230) involves heating the elution buffer in the wells (210) with the heating element (130) between the receptacles (110) on the upper face (105) of the heating adapter (120) to a predefined temperature and incubating the elution buffer and the magnetic particles (230), using conditions under which the biological target material is eluted from the surface of the magnetic particles (230).

13. The method of any of the claims 10 to 12, further comprising after step f) a step of adding a washing buffer to the wells for removing undesired components from the surface of the magnetic particles (230) while retaining the biological target material thereon, then withdrawing the liquid from the respective wells (210) while retaining the pellet (240) of magnetic particles (230) by magnetic force.

14. The method of claim 13, wherein washing of the magnetic particles (230) with the biological target material bound to their surface involves heating the suspension in the wells (210) with the heating element (130) between the receptacles (110) on the upper face (105) of the heating adapter (120) to a predefined temperature and incubating the suspension, using conditions under which the biological target material remains bound to the surface of the magnetic particles (230).

## Patentansprüche

1. System zum Verarbeiten eines biologischen Zielmaterials in einer flüssigen Probe (220), wobei das System Folgendes umfasst:
- eine Mikrotiterplatte (200) mit einer Vielzahl von Vertiefungen (210) mit einer offenen Oberseite und einem geschlossenen Boden, wobei zumindest ein Teil der Vielzahl von Vertiefungen (210) eine Suspension von Magnetpartikeln (230) mit einer Bindungsfläche umfasst;
- eine Magnetscheidevorrichtung (300), die eine Magnetscheideplatte (310) mit einer Vielzahl von Magneten (320) in einer vorbestimmten geometrischen Anordnung umfasst, wobei die Magnetscheidevorrichtung (300) ferner einen Aktuator (330) zum Bewegen der Magnetscheideplatte (310) relativ zur Mikrotiterplatte (200) umfasst;
- eine Heizvorrichtung (100), umfassend einen im Wesentlichen nicht-magnetischen Heizadapter (120) mit einer oberen Fläche (105), einer unteren Fläche (115) und einer Vielzahl von Aufnahmen (110), die zur oberen Fläche (105) offen sind, wobei die Aufnahmen (110) so geformt sind, dass sie die Vertiefungen (210) der Mikrotiterplatte (200) aufnehmen, und ein Heizelement (130) zwischen den Aufnahmen (110) an der oberen Fläche (105) des Heizadapters (120), wobei der Heizadapter (120) ferner an Stellen, die der vorbestimmten geometrischen Anordnung der Vielzahl von Magneten (320) der Magnetscheideplatte (310) entsprechen, Aussparungen (125), die zur unteren Fläche (115) offen sind, umfasst, wobei sich die Aufnahmen (110) und die Aussparungen (125) in ausreichender Nähe befinden, damit die eingeführten Magnete (320) einen Magnetfeldgradienten an die entsprechenden eingeführten Vertiefungen (210) anlegen können.

2. System nach Anspruch 1, ferner umfassend ein oder mehrere Elemente, ausgewählt aus der Gruppe von einer Pipettiervorrichtung (500), einer Steuereinheit und einer Datenverwaltungseinheit.

3. System nach einem der vorstehenden Ansprüche, wobei die Heizvorrichtung (100) ferner einen Temperaturfühler (140) umfasst.

4. System nach einem der vorstehenden Ansprüche, wobei das Heizelement (130) ein oder mehrere Element(e) umfasst, das/die aus der Gruppe, bestehend aus einem elektrisch leitfähigen Widerstandsmaterial und einem fluidischen oder mikrofluidischen System, das Wärmetransportmedien enthält, ausgewählt ist/sind.

5. System nach einem der vorstehenden Ansprüche, wobei das Heizelement (130) auf die Oberfläche des Heizadapters (120) gedruckt ist.

6. System nach einem der vorstehenden Ansprüche, wobei das Heizelement (130) auf die obere Fläche (105) des Heizadapters (120) gesputtert oder im Vakuum aufgedampft ist.

7. System nach einem der vorstehenden Ansprüche, wobei die Mikrotiterplatte (200) Abmessungen nach dem Standard-SBS-Format hat.

8. System nach einem der vorstehenden Ansprüche, wobei die Magnetscheidevorrichtung (300) ferner an ihrer oberen Fläche einen Rahmen (360) zur Aufnahme der Heizvorrichtung (100) und der Mikrotiterplatte (200) umfasst.

9. System nach einem der vorstehenden Ansprüche, wobei der Heizadapter (120) aus einem oder mehreren Materialien, ausgewählt aus der Gruppe, bestehend aus Aluminium und seinen Legierungen, Kupfer, Stahl, Silber, Aluminiumoxidkeramik und Siliciumcarbid, gefertigt ist.

10. Verfahren zum Verarbeiten eines biologischen Zielmaterials in einer flüssigen Probe (220), wobei das Verfahren die folgenden Schritte umfasst:
a) Bereitstellen einer Mikrotiterplatte (200) mit einer Vielzahl von Vertiefungen (210) mit einer offenen Oberseite und einem geschlossenen Boden;
b) Einführen der Vertiefungen (210) der Mikrotiterplatte (200) in entsprechende Aufnahmen (110) an der oberen Fläche (105) eines im Wesentlichen nicht-magnetischen Heizadapters (120), der aus einer Heizvorrichtung (100) besteht, wobei die Aufnahmen (110) so geformt sind, dass sie die Vertiefungen (210) der Mikrotiterplatte (200) aufnehmen;
c) Zugeben der flüssigen Probe (220) und einer Suspension von Magnetpartikeln (230) mit einer Bindungsfläche, entweder einzeln oder zusammen, in zumindest einen Teil der Vielzahl von Vertiefungen (210) während oder nach einem der Schritte a) oder b);
d) Erhitzen der flüssigen Probe (220) in den Vertiefungen (210) mit einem Heizelement (130) zwischen den Aufnahmen (110) an der oberen Fläche (105) des Heizadapters (120) auf eine vordefinierte Temperatur und Inkubieren der flüssigen Probe (220) und der Magnetpartikel (230) unter Anwendung von Bedingungen, unter denen das biologische Zielmaterial an die Oberfläche der Magnetpartikel (230) bindet;
e) Einbringen einer Vielzahl von Magneten (320) einer Magnetscheideplatte (300) in entsprechende Aussparungen (125) in einer unteren Fläche (115) des Heizadapters (120), wobei jeder Magnet (410) in ausreichende Nähe zur Außenwand einer entsprechenden Vertiefung (210) gebracht wird, damit durch den Heizadapter (120) ein Magnetfeldgradient an den Innenraum der Vertiefung (210) angelegt wird, wodurch ein Pellet (240) aus Magnetpartikeln (230) gebildet wird;
f) Abziehen der Flüssigkeit aus den jeweiligen Vertiefungen (210) mit einer Vielzahl von Pipettenspitzen (510) einer Pipettiervorrichtung (500), während das Pellet (240) aus Magnetpartikeln (230) durch Magnetkraft zurückgehalten wird.

11. Verfahren nach Anspruch 10, das ferner die folgenden Schritte umfasst:
g) Zugeben eines Elutionspuffers in die Vertiefungen (210) und Resuspendieren der Magnetpartikel (230) darin;
h) Eluieren des biologischen Zielmaterials von den Magnetpartikeln (230) mit dem Elutionspuffer.

12. Verfahren nach Anspruch 11, wobei die Elution des biologischen Zielmaterials von der Bindungsfläche der Magnetpartikel (230) das Erhitzen des Elutionspuffers in den Vertiefungen (210) mit dem Heizelement (130) zwischen den Aufnahmen (110) an der oberen Fläche (105) des Heizadapters (120) auf eine vordefinierte Temperatur und Inkubieren des Elutionspuffers und der Magnetpartikel (230) unter Anwendung von Bedingungen, unter denen das biologische Zielmaterial von der Oberfläche der Magnetpartikel (230) eluiert wird, einschließt.

13. Verfahren nach einem der Ansprüche 10 bis 12, ferner umfassend nach Schritt f) einen Schritt des Zugebens eines Waschpuffers in die Vertiefungen zum Entfernen unerwünschter Komponenten von der Oberfläche der Magnetpartikel (230), während das biologische Zielmaterial darauf zurückgehalten wird, dann Abziehen der Flüssigkeit aus den jeweiligen Vertiefungen (210), während das Pellet (240) aus Magnetpartikeln (230) durch Magnetkraft zurückgehalten wird.

14. Verfahren nach Anspruch 13, wobei das Waschen der Magnetpartikel (230) mit dem an ihre Oberfläche gebundenen biologischen Zielmaterial das Erhitzen der Suspension in den Vertiefungen (210) mit dem Heizelement (130) zwischen den Aufnahmen (110) an der oberen Fläche (105) des Heizadapters (120) auf eine vordefinierte Temperatur und Inkubieren der Suspension unter Anwendung von Bedingungen, unter denen das biologische Zielmaterial an der Oberfläche der Magnetpartikel (230) gebunden bleibt, einschließt.

## Revendications

1. Système pour le traitement d'une substance biologique cible dans un échantillon liquide (220), le système comprenant :
- une plaque multipuits (200) ayant une pluralité de puits (210) avec un sommet ouvert et un fond fermé, dans laquelle au moins une partie de la pluralité de puits (210) comprend une suspension de particules magnétiques (230) avec une surface de liaison ;
- un dispositif de séparation magnétique (300) comprenant une plaque de séparation magnétique (310) ayant une pluralité d'aimants (320) dans une configuration géométrique prédéterminée, le dispositif de séparation magnétique (300) comprenant en outre un actionneur (330) pour déplacer la plaque de séparation magnétique (310) par rapport à la plaque multipuits (200) ;
- un dispositif de chauffage (100) comprenant un adaptateur de chauffage (120) essentiellement non magnétique ayant une face supérieure (105), une face inférieure (115) et une pluralité de réceptacles (110) s'ouvrant vers la face supérieure (105), les réceptacles (110) étant façonnés pour recevoir les puits (210) de la plaque multipuits (200), et un élément chauffant (130) entre les réceptacles (110) sur la face supérieure (105) de l'adaptateur de chauffage (120), l'adaptateur de chauffage (120) comprenant en outre des évidements (125) s'ouvrant vers la face inférieure (115), dans des positions correspondant à la configuration géométrique prédéterminée de la pluralité d'aimants (320) de la plaque de séparation magnétique (310), dans lequel les réceptacles (110) et les évidements (125) sont suffisamment proches pour permettre aux aimants (320) insérés d'imposer un gradient de champ magnétique sur les puits (210) insérés correspondants.

2. Système selon la revendication 1, comprenant en outre un ou plusieurs éléments choisis dans le groupe d'un pipeteur (500), d'une unité de commande et d'une unité de gestion des données.

3. Système selon l'une quelconque des revendications précédentes, dans lequel le dispositif de chauffage (100) comprend en outre un capteur de température (140).

4. Système selon l'une quelconque des revendications précédentes, dans lequel l'élément chauffant (130) comprend un ou plusieurs éléments choisis dans le groupe constitué par un matériau résistif électriquement conducteur et un système fluidique ou microfluidique contenant un support caloporteur.

5. Système selon l'une quelconque des revendications précédentes, dans lequel l'élément chauffant (130) est imprimé sur la face supérieure de l'adaptateur de chauffage (120).

6. Système selon l'une quelconque des revendications précédentes, dans lequel l'élément chauffant (130) est pulvérisé ou déposé sous vide sur la face supérieure (105) de l'adaptateur de chauffage (120).

7. Système selon l'une quelconque des revendications précédentes, dans lequel la plaque multipuits (200) a des dimensions conformément au format SBS standard.

8. Système selon l'une quelconque des revendications précédentes, dans lequel le dispositif de séparation magnétique (300) comprend en outre sur sa face supérieure un cadre (360) pour recevoir le dispositif de chauffage (100) et la plaque multipuits (200).

9. Système selon l'une quelconque des revendications précédentes, dans lequel l'adaptateur de chauffage (120) est fabriqué en un ou plusieurs matériaux choisis dans le groupe constitué par l'aluminium et ses alliages, le cuivre, l'acier, l'argent, les céramiques d'alumine et le carbure de silicium.

10. Procédé pour le traitement d'une substance biologique cible dans un échantillon liquide (220), le procédé comprenant les étapes de :
a) fourniture d'une plaque multipuits (200) ayant une pluralité de puits (210) avec un sommet ouvert et un fond fermé ;
b) insertion des puits (210) de la plaque multipuits (200) dans les réceptacles (110) correspondants sur la face supérieure (105) d'un adaptateur de chauffage (120) essentiellement non magnétique inclus dans un dispositif de chauffage (100), les réceptacles (110) étant façonnés pour recevoir les puits (210) de la plaque multipuits (200) ;
c) ajout de l'échantillon liquide (220) et d'une suspension de particules magnétiques (230) avec une surface de liaison, soit individuellement, soit ensemble, à au moins une partie de la pluralité de puits (210) pendant, ou après l'une quelconque des étapes a) ou b) ;
d) chauffage de l'échantillon liquide (220) dans les puits (210) avec un élément chauffant (130) entre les réceptacles (110) sur la face supérieure (105) de l'adaptateur de chauffage (120) à une température prédéfinie et incubation de l'échantillon liquide (220) et des particules magnétiques (230), en utilisant des conditions sous lesquelles la substance biologique cible se lie à la surface des particules magnétiques (230) ;
e) introduction d'une pluralité d'aimants (320) d'une plaque de séparation magnétique (300) dans les évidements (125) correspondants d'une face inférieure (115) de l'adaptateur de chauffage (120), dans laquelle chaque aimant (410) est amené suffisamment proche de la paroi extérieure d'un puits (210) correspondant pour imposer un gradient de champ magnétique sur l'espace intérieur dudit puits (210) à travers l'adaptateur de chauffage (120), formant ainsi une bille (240) de particules magnétiques (230) ;
f) retrait du liquide des puits (210) respectifs avec une pluralité de pointes de pipettes (510) d'un pipeteur (500) tout en retenant la bille (240) de particules magnétiques (230) par force magnétique.

11. Procédé selon la revendication 10, comprenant en outre les étapes suivantes :
g) ajout d'un tampon d'élution aux puits (210) et resuspension des particules magnétiques (230) dans ceux-ci ;
h) élution de la substance biologique cible depuis les particules magnétiques (230) avec le tampon d'élution.

12. Procédé selon la revendication 11, dans lequel l'élution de la substance biologique cible depuis la surface de liaison des particules magnétiques (230) implique le chauffage du tampon d'élution dans les puits (210) avec l'élément chauffant (130) entre les réceptacles (110) sur la face supérieure (105) de l'adaptateur de chauffage (120) à une température prédéfinie et l'incubation du tampon d'élution et des particules magnétiques (230), en utilisant des conditions sous lesquelles la substance biologique cible est éluée depuis la surface des particules magnétiques (230).

13. Procédé selon l'une quelconque des revendications 10 à 12, comprenant en outre après l'étape f) une étape d'ajout d'un tampon de lavage aux puits pour éliminer les composants non souhaités de la surface des particules magnétiques (230) tout en retenant la substance biologique cible sur celles-ci, puis de retrait du liquide des puits (210) respectifs tout en retenant la bille (240) de particules magnétiques (230) par force magnétique.

14. Procédé selon la revendication 13, dans lequel le lavage des particules magnétiques (230) avec la substance biologique cible liée à leur surface implique le chauffage de la suspension dans les puits (210) avec l'élément chauffant (130) entre les réceptacles (110) sur la face supérieure (105) de l'adaptateur de chauffage (120) à une température prédéfinie et l'incubation de la suspension, en utilisant des conditions sous lesquelles la substance biologique cible reste liée à la surface des particules magnétiques (230).
